# EUROPEAN PATENT SPECIFICATION

(11) **EP 4 225 952 B1**
(45) Date of publication and mention of the grant of the patent: **09.04.2025**
(21) Application number: 22790219.4
(22) Date of filing: 09.09.2022
(51) Int. Cl.: C12Q 1/689, C12Q 1/70

(54) **COMPOSITIONS, KITS, METHODS FOR DETECTING AND IDENTIFYING PATHOGENS THAT CAUSE RESPIRATORY TRACT INFECTIONS AND USE THEREOF**
ZUSAMMENSETZUNGEN, KITS, VERFAHREN ZUM NACHWEIS UND ZUR IDENTIFIZIERUNG VON PATHOGENEN, DIE ATEMWEGSINFEKTIONEN VERURSACHEN, UND VERWENDUNG DAVON
COMPOSITIONS, KITS, PROCÉDÉS DE DÉTECTION ET D'IDENTIFICATION D'AGENTS PATHOGÈNES PROVOQUANT DES INFECTIONS DES VOIES RESPIRATOIRES ET LEUR UTILISATION

(30) Priority: 16.11.2021 CN 202111356118
(43) Date of publication of application: 16.08.2023
(73) Proprietor: Sansure Biotech Inc., Changsha, Hunan 410205 (CN)
(72) Inventor: DAI, Lizhong, Hunan 410205 (CN); CHEN, Xiaoliang, Hunan 410205 (CN); REN, Xiaomei, Hunan 410205 (CN); DENG, Zhongping, Hunan 410205 (CN); LIU, Jia, Hunan 410205 (CN)
(74) Representative: Grünecker Patent- und Rechtsanwälte PartG mbB
(86) International application number: PCT/CN2022/117972
(87) International publication number: WO 2023/087868

(56) References cited:
- WO-A1-2013/049891
- CN-A- 111 074 001
- CN-A- 113 943 836
- BRITTAIN-LONG ET AL: "Multiplex real-time PCR for detection of respiratory tract infections", JOURNAL OF CLINICAL VIROLOGY, ELSEVIER, AMSTERDAM, NL, vol. 41, no. 1, 26 December 2007 (2007-12-26), pages 53 - 56, XP022401293, ISSN: 1386-6532

## Description

### TECHNICAL FIELD

The present application relates to the field of molecular biological detection, specifically, to the field of detection of pathogens that cause respiratory tract infections. More specifically, the present disclosure is capable of simultaneously detecting influenza A, influenza B, rhinovirus, respiratory adenovirus, and respiratory syncytial virus and mycoplasma pneumoniae.

### BACKGROUND

Infectious respiratory system diseases are the most common and frequently-occurring diseases in clinical practice. These diseases have similar clinical symptoms and epidemiological characteristics, and thus is difficult to be identified and determined for the pathogen species infected according to clinical symptoms and routine laboratory tests. Respiratory pathogens can be transmitted through the air. The pathogens that cause acute respiratory diseases have the characteristics of strong infectivity, rapid spread, short incubation period, and acute onset, and may cause a wide range of acute diseases of upper and lower respiratory tract, which seriously endanger human health. Common respiratory pathogens include the followings.

Influenza virus, also referred to as flu virus, is an RNA virus that causes influenza in humans and animals. Human influenza viruses, classified into three types of A, B, and C, are the pathogens of influenza (flu). Avian influenza (AI), also known as bird flu, is an infectious disease caused by a subtype of influenza A (also known as avian influenza virus). Taxonomically, the influenza virus belongs to the Influenza virus genus in the family Orthomyxoviridae. It can cause acute upper respiratory tract infection and spread rapidly through the air, and often leads to periodic pandemics that occur globally, such as the "Spanish Influenza" that killed more than 20 million people worldwide in 1918-1919, the "Asian Influenza" that occurred in 1957, and the "Hong Kong Influenza" that occurred in 1968 and the "Russian Influenza" occurred in 1977, as well as the H7N9 avian influenza occurred in 2013.

Respiratory syncytial virus (RSV) is an RNA virus belonging to the Pneumonia genus in the family Paramyxoviridae. Clinical studies have shown that RSV is the most common pathogen causing viral pneumonia. RSV infection is more common in infants under three years of age, with the main symptoms being high fever, rhinitis, pharyngitis and laryngitis, and later manifesting as bronchiolitis and pneumonia, and may be complicated by otitis media, pleurisy and myocarditis, etc. in a small number of sick children. Infection in adults and older children mainly manifests upper respiratory tract infections.

Adenovirus (Adv) belongs to the family Adenoviridae. According to their different immunological, biological and biochemical characteristics, adenovirus is classified into 7 subspecies from A to G, with a total of 52 serotypes. Different serotypes have different organ affinities and cause corresponding clinical manifestations, and may infect the respiratory tract, gastrointestinal tract, urethra, bladder, eyes, liver, and the like.

Human rhinovirus (HRV) is a non-enveloped, single-stranded RNA virus belonging to the genus Enterovirus in the Picornaviridae family. Human rhinovirus has the same morphological structure and genome structure as enterovirus. It forms a spherical shape with a diameter of 15-30 nm, and has a nucleocapsid with icosahedral symmetry and no envelope. The genome is single-stranded, positive-strand RNA that is about 7500 nucleotides in length, and includes only one reading frame that is about 6500 nucleotides in length; the reading frame is initiated 611 nucleotides from 5' end, and stops 42 nucleotides from the poly A tail. Human rhinovirus is a highly contagious and ubiquitous virus that is usually transmitted through direct contact with respiratory droplets/microdroplets, and also through contaminated surfaces, including person-to-person direct contact. HRV has three distinct subgroups A, B, and C consisting of 80, 30, and 56 types, respectively. It is the most serotyped virus among human infectious viruses. HRV's genetic diversity (>150 types) makes it very difficult to develop an effective vaccine. A certain degree of immune protection may be gained after infection, however, previous infection cannot provide complete immunity due to too many serotypes of HRV, that is to say, other serotypes may still lead to reinfection. HRV is the leading cause of the "common cold" manifesting as rhinorrhea, sore throat, cough, and malaise. HRV also causes many other respiratory diseases, such as asthma, chronic obstructive pulmonary disease, otitis media, angina, pneumonia and acute bronchiolitis. HRV co-infection with other respiratory viruses, more commonly respiratory syncytial virus, adenovirus, and the like, occurs frequently and aggravate the patient's condition, seriously affecting the life quality of the patients.

Mycoplasma pneumonia (MP) is a common pathogen of community-acquired pneumonia. Infection with MP can cause atypical pneumonias with pathological changes mainly manifesting as interstitial pneumonia, sometimes complicated by bronchial pneumonia. MP is mainly transmitted through droplets, has an incubation period of 2 to 3 weeks and presents the highest incidence in adolescents. MP causes mild clinical symptoms with only general respiratory symptoms such as headache, sore throat, fever and cough in most patients, and a few would have a persistent high fever, severe cough, and a rapidly progressing disease, leading to severe or critical conditions such as respiratory failure, multiple organ dysfunction in a short period of time.

Conventionally, some technologies are used for detecting respiratory tract infection pathogens, such as Chinese invention patent CN113186342A, which can detect 18 kinds of pathogens; CN111074001A, which can detect 9 kinds of pathogens. However, these techniques still face the problems of poor detectable rate and accuracy due to the complexity of the pathogens to be detected.

Therefore, there is a need in the art for a detection kit for rapidly and accurately detecting the most common respiratory tract infection pathogens with a high detectable rate, accuracy and sensitivity.

### SUMMARY

Accordingly, the detection of various viruses is required to be specifically configured in order to improve the detectable rate and accuracy. Taking the nucleic acid detection for rhinovirus as an example, since both human rhinovirus and enterovirus belong to the genus Enterovirus, many reagents or methods for the detection cannot distinguish between human rhinovirus and enterovirus, or can distinguish them but with poor specificity. The reason why these reagents cannot accurately detect rhinovirus is that only the conservative property of 5' untranslated region of the genus Enterovirus is considered when designing primers and probes, so rhinovirus cannot be well differentiated from enterovirus. Against gene sequences of different mutation types of rhinovirus, the inventors have designed two probes that can cover various mutation types of rhinovirus without non-specific binding to enterovirus. By using such two probes with completely different sequences but the same fluorescent labels, the rhinovirus can be detected accurately and effectively.

Taking the detection of respiratory adenovirus as an example, two forward primers and two identical fluorescence-labeled probes are designed for various genotypes of adenovirus in order to ensure the accuracy of the detection results, since there are 52 serotypes of adenovirus among which types 3, 7, 11, 14, 16, 21, 34, 35, 50 and 55, etc. capable of causing respiratory infections in humans and recombination of 2 or more adenovirus strains can produce a new type of adenovirus.

In a first aspect, the present application provides a composition for detecting and identifying a pathogen that causes a respiratory tract infection, the composition including:
a first nucleic acid composition, including:
   an influenza A forward primer as set forth in SEQ ID NO:1, an influenza A reverse primer as set forth in SEQ ID NO:2, and an influenza A probe as set forth in SEQ ID NO:3;
   an influenza B forward primer as set forth in SEQ ID NO:4, an influenza B reverse primer as set forth in SEQ ID NO:5, and an influenza B probe as set forth in SEQ ID NO:6;
   a rhinovirus forward primer as set forth in SEQ ID NO:7, a rhinovirus reverse primer as set forth in SEQ ID NO:8, a rhinovirus probe as set forth in SEQ ID NO:9, and a rhinovirus probe as set forth in SEQ ID NO:10; and
   a second nucleic acid composition, including:
      a respiratory adenovirus forward primer as set forth in SEQ ID NO: 11, a respiratory adenovirus forward primer as set forth in SEQ ID NO: 12, a respiratory adenovirus reverse primer as set forth in SEQ ID NO: 13, a respiratory adenovirus probe as set forth in SEQ ID NO: 14, and a respiratory adenovirus probe as set forth in SEQ ID NO: 15;
      a respiratory syncytial virus forward primer as set forth in SEQ ID NO:16, a respiratory syncytial virus reverse primer as set forth in SEQ ID NO:17, and a respiratory syncytial virus probe as set forth in SEQ ID NO: 18;
      a mycoplasma pneumoniae forward primer as set forth in SEQ ID NO:19, a mycoplasma pneumoniae reverse primer as set forth in SEQ ID NO:20, and a mycoplasma pneumoniae probe as set forth in SEQ ID NO:21;
      wherein, fluorescent groups comprised in the respective probes of the first nucleic acid composition are different from each other and do not interfere with each other, and fluorescent groups comprised in the respective probes of the second nucleic acid composition are different from each other and do not interfere with each other.

The expression "different from each other and do not interfere with each other" herein means that the fluorescent groups used by respective probe in the composition are different, and will not affect the detection of each other, that is, can be detected using different. For example, FAM, HEX, ROX and CY5 may be used. These groups that do not have approximate absorbance values allow the selection of different channels, and thus will not interfere with each other.

In a further embodiment, the composition includes an internal standard forward primer, an internal standard reverse primer and an internal standard probe, that are configured for monitoring.

In one specific embodiment, the composition further includes an internal standard forward primer as set forth in SEQ ID NO: 22, an internal standard reverse primer as set forth in SEQ ID NO: 23, and an internal standard probe as set forth in SEQ ID NO: 24.

In the present application, the fluorescent reporter group may be selected from a group consisting of FAM, HEX, ROX, VIC, CY5, 5-TAMRA, TET, CY3 and JOE, but is not limited thereto

In one specific embodiment, the fluorescent reporter group of the influenza A probe as set forth in SEQ ID NO:3 is FAM; the fluorescent reporter group of the influenza B probe as set forth in SEQ ID NO:6 is HEX; the fluorescent reporter group of the human rhinovirus probe as set forth in SEQ ID NOs: 9 and 10 is CY5.

In one specific embodiment, the fluorescent reporter group of the respiratory adenovirus probe as set forth in SEQ ID Nos: 14 and 15 is FAM; the fluorescent reporter group of the respiratory syncytial virus probe as set forth in SEQ ID NO: 18 is HEX; the fluorescent reporter group of the mycoplasma pneumoniae probe as set forth in SEQ ID NO· 21 is CY5

In one specific embodiment, the fluorescent reporter group of the internal standard probe as set forth in SEQ ID NO: 24 is ROX.

The internal standard is the primers and probe that detect human housekeeping gene GAPDH gene sequence. GAPDH gene sequence (SEQ ID NO: 25) is:

GGGGAGCCAAAAGGGTCATCATCTCTGCCCCCTCTGCTGATGCCCCCA
TGTTCGTCATGGGTGTGAACCATGAGAAGTATGACAACAGCC

In a further embodiment, the detection primers are used in the composition in an amount of 0.2-0.4 pM; the detection probes are used in the composition in an amount of 0.1-0.3 pM; the internal standard primers are used in the composition in an amount of 0.2 pM; and the internal standard probe is used in the composition in an amount of 0.1 pM.

In the present application, the terms "detection primers" and "detection probes" refer to the primers and probes for amplification and detection of a pathogen.

In the present application, the terms "internal standard primer" and "internal standard probe" refer to a primer and a probe for amplification and detection of an internal standard.

In a further specific embodiment, each component in each nucleic acid composition of the compositions of the present application is present in a mixed form.

In a second aspect, the present application provides use of the above-mentioned composition of the present application in the preparation of a kit for detecting and identifying a pathogen that causes a respiratory tract infection.

The pathogen that causes a respiratory tract infection includes influenza A, influenza B, respiratory adenovirus, human rhinovirus, respiratory syncytial virus and mycoplasma pneumoniae.

In a third aspect, the present application provides a kit for detecting and identifying a pathogen that causes a respiratory tract infection, the kit comprising the first nucleic acid composition and the second nucleic acid composition of the present invention in separate packages.

In a further embodiment, the kit further includes a nucleic acid release reagent and a PCR amplification system.

In a further embodiment, the kit also includes at least one of a nucleic acid release reagent, dNTP, reverse transcriptase, DNA polymerase, a PCR buffer and Mg²⁺.

Common PCR buffers include Tris-HCl, MgCl₂, KCl, Triton X-100 and other buffer systems. Generally, a total volume in a single PCR reaction tube is 20~200µl.

In a further embodiment, the detection primers are used in the composition in an amount of 25-500 nM; the detection probes are used in the composition in an amount of 20-500 nM; the internal standard primers are used in the composition in an amount of 12.5~250 nM; the internal standard probe is used in the composition in an amount of 10~250 nM; and dNTP are used is in an amount of 0.2-0.3 mM.

In further embodiment, the reverse transcriptase is at a concentration of 5 U/µL to 15 U/ul., and the reverse transcriptase may be, for example, murine leukemia reverse transcriptase (MMLV) or Tth enzyme. The DNA polymerase is at a concentration of 5 U/µL to 15 U/µL, and the DNA polymerase may be, for example, Taq enzyme.

In a further embodiment, the kit contains a positive control and a negative control. The negative and positive controls need to be processed simultaneously with the sample to be tested. The positive control, which simulates actual clinical specimens, is a mixture of artificially synthesized lentiviral particles containing specific nucleic acid sequences of influenza A, influenza B, human rhinovirus and respiratory syncytial virus, and artificially synthesized plasmids containing specific nucleic acid sequences of adenovirus and mycoplasma pneumoniae. The negative control, which completely simulates throat swab samples of normal people, is composed of artificially synthesized lentiviral particles containing the target fragment (SEQ ID NO: 25) of GAPDH housekeeping gene.

In a fourth aspect, a method for detecting and identifying a pathogen that causes a respiratory tract infection is provided. The method includes the following steps:
1) releasing a nucleic acid in a sample to be detected;
2) performing a fluorescence quantitative PCR on the nucleic acid obtained in step 1) using the above-mentioned composition or the above-mentioned kit of the present application; and
3) obtaining a result and analyzing the result.

In the present application, the sample to be tested may be a throat swab, sputum, bronchoalveolar lavage fluid, blood, etc., but is not limited thereto.

In a further embodiment, the fluorescent quantitative PCR is performed under the following conditions:
1 cycle of pre-denaturation and enzyme activation at a temperature of 95°C for 1 to 10 minutes;1 cycle of reverse transcription at a temperature of 60°C for 25 to 35 minutes; 1 cycle of cDNA pre-denaturation at a temperature of 95°C for 1 to 10 minute; 40 to 50 cycles of denaturation at a temperature of 95°C for 10 to 20 seconds and annealing at a temperature of 60°C for 20 to 40 seconds.

In one specific embodiment, a method for detecting and identifying a pathogen that causes a respiratory tract infection for non-diagnostic purposes is provided. The method includes the following steps:
1) releasing a nucleic acid in a sample to be detected;
2) performing a fluorescence quantitative PCR on the nucleic acid obtained in step 1) using the above-mentioned composition or the above-mentioned kit of the present application; and
3) obtaining a result and analyzing the result.

The compositions of the present application can be used to simultaneously detect six pathogens that cause respiratory tract infection, leading to simple, quick, and objective detection of the respiratory tract pathogens, thereby realizing identification of pathogens, early diagnosis, rational guidance of clinical medication, reduced waste of medical resources, and reduced psychological burden on patients and society. At the same time, it has a higher detectable rate than that of the existing respiratory pathogen detection composition, and is more accurate in detection.

The composition of the present application, combined with the fluorescent probe method, enables the simultaneous use of two tubes in one test, and thus has low cost and high throughput. The information of four target points can be given in one tube in a single test with simple and convenient operation, and the results can be read by judgment with CT values. The whole process of the detection is carried out in a single tube under sealing, which avoids false positives and environmental contamination caused by cross-over between samples.

### BRIEF DESCRIPTION OF THE DRAWINGS

Fig. 1 is a graph showing the result of the detection using the composition of the present application.
Figs. 2-7 are graphs showing the sensitivity of the compositions of the present application.
Figs. 8-9 are graphs showing the specificity of the compositions of the present application.
Fig. 10A is a graph showing the effect of the compositions of the present application, and Fig. 10B is a graph showing the effect when the respiratory syncytial virus in the present application is replaced by SARS-CoV-2.
Fig. 11A is a graph showing the effect of the compositions of the present application, and Fig. 11B is a graph showing the effect when the rhinovirus of the present application is replaced by metapneumovirus.

### DETAILED DESCRIPTION OF THE EMBODIMENTS

Hereinafter, the present application will be described in detail with reference to specific embodiments and examples, and the advantages and various effects of the present application will be more clearly presented therefrom. It is understood by those skilled in the art that these specific embodiments and examples are intended to illustrate, but not to limit the present application.

### Example 1. Primers and probes used in the present application

The primers and probes used in the present application are shown in Table 1 below (SEQ ID NOs: 1 to 24 corresponds to the base sequences only, and the labeling modes here are shown as specific examples only):

**Table 1**

| Name | Detection target | Sequences and labeling modes (5'-3') |
|---|---|---|
| SEQ ID NO:1 | Influenza A | TCTCATGGAATGGCTAAAGACAA |
| SEQ ID NO:2 | | AGGGCATTTTGGACAAAGC |
| SEQ ID NO:3 | | FAM-CACCGTGCCCAGTGAGCGAGGA-BHQ1 |
| SEQ ID NO:4 | Influenza B | TCACTCTTCGAGCGTTTTAATGAAG |
| SEQ ID NO:5 | | TAATCGGTGCTCTTGACCAAAT |
| SEQ ID NO: 6 | | HEX-AGCCAATTCGAGCAGCTGAAACTGCG- BHQ1 |
| SEQ ID NO:7 | Human Rhinovirus | GCCCCTGAATGCGGCTAA |
| SEQ ID NO:8 | | GAAACACGGACACCCAAAGTAGT |
| SEQ ID NO:9 | | CY5-TAATGAGCAATTGCGGGATGGGACC- BHQ2 |
| SEQ ID NO:10 | | CY5- CTAGTGCATACAATCCAGTGTGTRGCTAG- BHQ2 |
| SEQ ID NO:11 | Respiratory Adenovirus | GGATCCACCTCAAAAGTCAT |
| SEQ ID NO: 12 | | TGGGCGGAGTTGGCGTAGA |
| SEQ ID NO:13 | | GTTAAGAGTATTACCCAGAAAAAGTT |
| SEQ ID NO:14 | | FAM-ACATGAAGTTGCTGGAGAAGGGGAT- BHQ1 |
| SEQ ID NO:15 | | FAM- CCCATGGACATAAAGTTACTGGAGAAGG- BHQ1 |
| SEQ ID NO:16 | Respiratory Syncytial Virus | AGAACAAGATGGGGCAAATATG |
| SEQ ID NO:17 | | TATGTTGATGCTTGCAAGTTCTTTTAT |
| SEQ ID NO:18 | | HEX- CGTGAACAAGCTTCACGAAGGCTCCACA- BHQ1 |
| SEQ ID NO:19 | Mycoplasm a Pneumonia e | CCGTTTCTCCACCGGGTT |
| SEQ ID NO:20 | | GTTTTAGGCGCGGTTATATCATC |
| SEQ ID NO:21 | | CY5- CCCTGGATTGGGAATGGGTACAGGTATG- BHQ2 |
| SEQ ID NO:22 | Human Housekeepi ng Gene Internal Standard | GGGGAGCCAAAAGGGTCAT |
| SEQ ID NO:23 | | GGCTGTTGTCATACTTCTCATGGTT |
| SEQ ID NO:24 | | ROX-ATCTCTGCCCCCTCTGCTGATGCCC- BHQ2 |

### Example 2. Methods for detecting pathogens that cause respiratory tract infections and identifying the pathogens

In the present application, the samples to be detected were throat swabs, sputum and bronchoalveolar lavage fluid, and blood. Viral nucleic acid was extracted using the magnetic bead method. The following operation was performed in a sample processing chamber.

2.1. An appropriate number of 1.5 mL sterilized centrifuge tubes were labeled for negative control, positive control and samples to be tested, and 300 µL of RNA extraction solution 1 was added to each tube;

2.2. 200 µL of the sample to be tested, negative control or positive control was added per tube; and the tubes were capped, mixed by shaking for 10 seconds, and subjected to a short spin;

2.3. 100 µL of RNA extraction solution 2-mix (which should be mixed well before pipetted) was added to each tube, and the tubes were mixed by shaking for 10 seconds, then left standing at room temperature for 10 minutes;

2.4. After a short spin, the centrifuge tubes were placed on a separator for 3 minutes, and the solution was gently pipetted out (be careful not to touch the brown substance adsorbed on the tube wall);

2.5 600 µL of RNA extraction solution 3 and 200 µL of RNA extraction solution 4 were added to each tube, and the centrifuge tubes were mixed by shaking for 5 seconds, subjected to a short spin, and placed on the separator again;

2.6. After about 3 minutes, the supernatant was divided into two layers, a pipette tip was inserted into the bottom of the centrifuge tube to gently pipette the liquid out from the bottom completely and discard the liquid, and then the tube was left standing for 1 minute, and then the residual liquid at the bottom of the tube was pipetted out completely and discarded.

2.7. Elution was performed by adding 50µL of TE buffer (pH8.0) to each tube, then all the brown mixture obtained after elution was transferred into a 0.2mL of PCR reaction tube as a sample to be tested, and then the tubes were capped, and transferred to an amplification detection area.

A real-time fluorescent PCR reaction system was configured as follows:

### PCR reaction system A:

| Components | Volume/Concentration in each reaction |
|---|---|
| PCR buffer | 19.20 µL |
| dNTP (100mM) | 0.50 µL |
| 1mol/L MgCl₂ | 0.20 µL |
| Primer SEQ ID NO:1 (50 pmol/µL) | 0.40 µL |
| Primer SEQ ID NO:2 (50 pmol/µL) | 0.40 µL |
| Primer SEQ ID NO:4 (50 pmol/µL) | 0.40 µL |
| Primer SEQ ID NO:5 (50 pmol/µL) | 0.40 µL |
| Primer SEQ ID NO:7 (50 pmol/µL) | 0.30 µL |
| Primer SEQ ID NO:8 (50 pmol/µL) | 0.30 µL |
| Primer SEQ ID NO:22 (50 pmol/µL) | 0.2 µL |
| Primer SEQ ID NO:23 (50 pmol/µL) | 0.2 µL |
| Probe SEQ ID NO:3 (50 pmol/µL) | 0.20 µL |
| Probe SEQ ID NO:6 (50 pmol/µL) | 0.30 µL |
| Probe SEQ ID NO:9 (50 pmol/µL) | 0.20 µL |
| Probe SEQ ID NO:10 (50 pmol/µL) | 0.10 µL |
| Probe SEQ ID NO:24 (50 pmol/µL) | 0.10 µL |
| RNasin | 0.125 µL |
| 0.1% DEPC water | Make up to 43. 5 µL |

### PCR reaction system B:

| Component | Volume/Concentration in each reaction |
|---|---|
| PCR buffer | 19.20 µL |
| dNTP(100mM) | 0.5 µL |
| 1mol/L MgCl12 | 0.20 µL |
| Primer SEQ ID NO:11 (50 pmol/µL) | 0.40 µL |
| Primer SEQ ID NO:12 (50 pmol/µL) | 0.20 µL |
| Primer SEQ ID NO:13 (50 pmol/µL) | 0.40 µL |
| Primer SEQ ID NO:16 (50 pmol/µL) | 0.30 µL |
| Primer SEQ ID NO:17 (50 pmol/µL) | 0.30 µL |
| Primer SEQ ID NO:19 (50 pmol/µL) | 0.30 µL |
| Primer SEQ ID NO:20 (50 pmol/µL) | 0.30 µL |
| Primer SEQ ID NO:22 (50 pmol/µL) | 0.20 µL |
| Primer SEQ ID NO:23 (50 pmol/µL) | 0.20 µL |
| Probe SEQ ID NO:14 (50 pmol/µL) | 0.20 µL |
| Probe SEQ ID NO:15 (50 pmol/µL) | 0.10 µL |
| Probe SEQ ID NO:18 (50 pmol/µL) | 0.20 µL |
| Probe SEQ ID NO:21 (50 pmol/µL) | 0.20 µL |
| Probe SEQ ID NO:24 (50 pmol/µL) | 0.10 µL |
| RNasin | 0.125 µL |
| Sterilized purified water | Make up to 43.5 µL |

An enzyme mix consisting of Neoscript RT reverse transcriptase and H - Taq enzyme was obtained by mixing H-Taq enzyme (15 U/µL) and Neoscript RT enzyme (18 U/µL) according to a certain ratio (1 µL of H-Taq enzyme and 0.5 µL of Neoscript RT enzyme mix per person).

### A PCR amplification program was set up as follows:

| Step | | Temperature | Time | Cycle Number |
|---|---|---|---|---|
| 1 | reverse transcription | 50°C | 30 minutes | 1 |
| 2 | pre-denaturation and enzyme activation | 95°C | 1 minute | 1 |
| 3 | denaturation | 95°C | 15 seconds | 45 |
| | annealing, extension and fluorescence acquisition | 60°C | 30 seconds | |
| 4 | instrument cooling | 25°C | 10 seconds | 1 |

### Result analysis:

1) the detection signal for the target was FAM, HEX (or VIC) and CY5, and the detection signal for internal reference was ROX;
2) Baseline setting: Baseline was generally set to 3-15 cycles, which could be adjusted according to the actual situation. The adjustment principle was to select a region where fluorescence signal is relatively stable before the exponential amplification part, with a start point (Start) avoiding the signal fluctuation in the initial stage of fluorescence acquisition, and an end point (End) being less by 1-2 cycles than the Ct of a sample showing the earliest exponential amplification. Threshold setting: the setting principle was to make a threshold line just exceeded the highest point of a normal negative control.

A sample was confirmed positive if obvious sigmoid amplification curves occurred in FAM, HEX and CY5 channels and the Ct value was less than or equal to 40; and a sample was confirmed negative if no amplification curve occurs in FAM, HEX and CY5 channels (i.e., No Ct) or the Ct value was greater than 40, and the ROX internal standard channel was positive (Ct value ≤ 40). The details were as follows:

| Amplificati on results | FAM channel | | HEX channel | | CY5 channel | | ROX channel | |
|---|---|---|---|---|---|---|---|---|
| | Ct value ≤ 40 | No Ct or Ct value > 40 | Ct value ≤ 40 | No Ct or Ct value > 40 | Ct value ≤ 40 | No Ct or Ct value > 40 | Ct value ≤ 40 | No Ct or Ct value > 40 |
| reaction solution | | | | | | | | |
| PCR mix A | Flu A **positi ve** | Flu A negati ve | Flu B **positi ve** | Flu B negative | HRV **positi ve** | HRV negativ e | Intern al Stand | Intern al Stand |
| PCR mix B | ADV p**ositi ve** | ADV negati ve | RSV **positi ve** | RSV negative | MP **positi ve** | MP negativ e | ard **positi ve** | ard negati ve |

### Example 3. Detectable rate of the compositions of the present application

The detection was carried out using the compositions in Table 1 according to the method described in Example 2 of the present application, and a series of controls (other dual-probe schemes: probe 1: CY5-CTAACCTTAACCCCGCAGC-BHQ2; probe 2: CY5-AATCCTAACCATGGAGCAAG- BHQ2) were also designed for the detection as well. The detection results are shown in Tables 2 and 3.

**Table 2. Statistical table of Ct value results of different rhinovirus detection schemes**

| Sample number | Scheme of the present application | Other dual-probe schemes | Single-probe scheme | Sequencing results of 5' untranslated region |
|---|---|---|---|---|
| 1 | 13.88 | 26.13 | 17.76 | Rhinovirus A |
| 2 | 22.81 | 34.03 | 32.22 | Rhinovirus A39 |
| 3 | 31.40 | 40.47 | NoCt | Rhinovirus A |
| 4 | NoCt | 31.82 | 23.9 | Coxsackievirus EV71 |
| 5 | 29.03 | 33.38 | NoCt | Rhinovirus C |
| 6 | 22.33 | 31.62 | 24.79 | Rhinovirus A22 |
| 7 | 39.89 | NoCt | NoCt | Rhinovirus A22 |
| 8 | 19.11 | 28.55 | 21.21 | Rhinovirus A22 |
| 9 | 21.66 | 32.13 | NoCt | Rhinovirus A |
| 10 | 19.18 | 24.28 | 21.8 | Rhinovirus A |
| 11 | 39.67 | NoCt | NoCt | Rhinovirus A |
| 12 | 39.68 | 37.64 | NoCt | Rhinovirus A |
| 13 | 31.46 | 38.04 | NoCt | Rhinovirus A10 |
| 14 | 26.90 | NoCt | NoCt | Rhinovirus A |
| 15 | 24.28 | 32.12 | 28.19 | Rhinovirus A46 |
| 16 | 19.66 | 30.85 | 23.33 | Rhinovirus A103 |
| 17 | 23.78 | 27.22 | 25.31 | Rhinovirus A |
| 18 | 25.71 | 36.52 | NoCt | Rhinovirus A |
| 19 | 24.18 | 30.43 | 26.32 | Rhinovirus A22 |
| 20 | NoCt | NoCt | 30.79 | Coxsackievirus A6 |

| | | | | |
|---|---|---|---|---|
| Notes: a larger Ct value means a worse detection effect, and No Ct means negative. | | | | |

It could be seen from the detection results in the above table that, enteroviruses (Coxsackievirus EV71, Coxsackievirus A6) would not be detected by using the scheme of the present application; while for rhinovirus positive samples, Ct values obtained by detecting with the scheme of the present application were smaller, indicating that it has better detection effect than that of the other dual-probe scheme and single-probe scheme.

**Table 3. Summary of Ct value results of different adenovirus detection schemes**

| Sample Number | The Present Application Scheme | The present application scheme without SEQ ID NO: 12 | The present application scheme without SEQ ID NO:15 | The present application scheme without SEQ ID NO: 12 and SEQ ID NO: 15 |
|---|---|---|---|---|
| 1 | 31.08 | 29.17 | 41.27 | NoCt |
| 2 | 33.16 | NoCt | NoCt | NoCt |
| 3 | 34.92 | 33.89 | NoCt | NoCt |
| 4 | 34.56 | 34.84 | NoCt | NoCt |
| 5 | 25.89 | 37.27 | NoCt- | NoCt |
| 6 | 34.36 | NoCt | NoCt | NoCt |
| 7 | 21.55 | 21.72 | 21.48 | 32.72 |
| 8 | 20.37 | 20.45 | 20.78 | 40.89 |
| 9 | 34.29 | 33.79 | NoCt | NoCt |
| 10 | 36.09 | 36.24 | 33.16 | NoCt |
| 11 | 20.51 | 20.63 | 20.97 | NoCt |
| 12 | 23.74 | 23.70 | 23.94 | 34.04 |
| 13 | 22.45 | 22.63 | 22.95 | NoCt |
| 14 | 35.43 | 35.24 | 35.25 | NoCt |
| 15 | 26.84 | 26.70 | 27.83 | 37.85 |
| 16 | 34.78 | 34.24 | 34.65 | NoCt |
| 17 | 34.20 | 33.59 | 33.25 | 38.15 |
| 18 | 23.01 | 22.33 | 23.25 | 26.09 |
| 19 | 31.62 | 31.47 | 32.08 | 32.38 |
| 20 | 30.65 | 29.76 | 31.17 | 31.84 |
| 21 | 28.43 | 29.61 | 28.11 | 29.42 |
| 22 | 30.29 | 30.82 | 30.26 | 33.56 |

| | | | | |
|---|---|---|---|---|
| Note: a larger Ct value means a worse detection effect, and No Ct means negative. | | | | |

It could be seen from the detection results in the above table that, all 22 positive samples could be detected by using the compositions of the present application; where there was no primer SEQ ID NO: 12, 2 positive samples were not detected; where there was no probe SEQ ID NO: 12, 6 positive samples were not detected; where there was neither SEQ ID NO: 12 nor SEQ ID NO: 15, 13 positive samples were not detected.

### Example 4. Sensitivity of the compositions of the present application

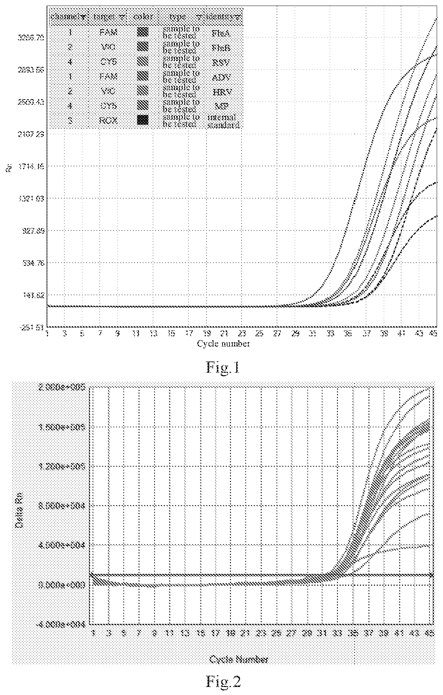

Six pathogens were tested using the compositions of the present application, and the detection results are shown in FIG. 1, indicating that the compositions of the present application were capable of detecting six pathogens.

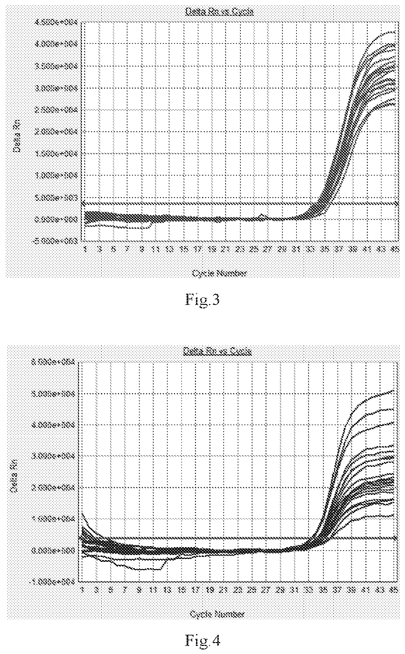

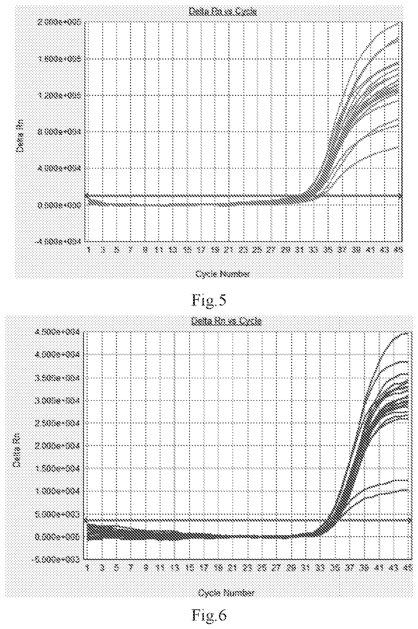

In addition, experiments were carried out with samples at different concentrations to detect the compositions of the present application for sensitivity using the compositions in Table 1 according to the method described in Example 2 of the present application. The positive samples for each target sensitivity were diluted in detection gradient, and the samples diluted to the detection limit were used as the test samples for detection, and each channel was detected 20 times. The analysis of sensitivity test results showed that, detection limits of this kit for influenza A, influenza B, human rhinovirus, respiratory syncytial virus, adenovirus and mycoplasma pneumoniae were respectively: 2.0 TCID50/mL, 2.0 TCID50/mL, 500.0 copies/mL, 500.0 copies/mL, 500.0 copies/mL, 500.0 copies/mL, as shown in Figs. 2-7.

### Example 5. Specificity of the compositions of the present application

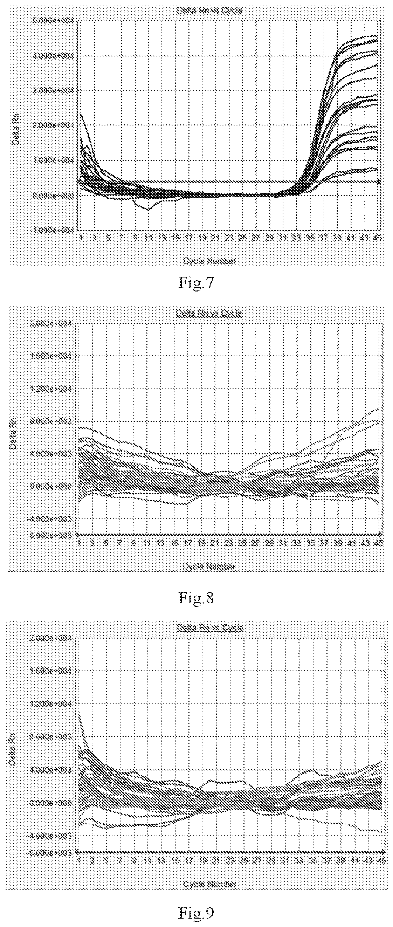

The compositions of the application invention were used to detect other common respiratory pathogens. The experiment showed that the compositions of the present application had no cross-reactivity with common respiratory pathogens (measles virus, mumps virus, rubella virus, *Staphylococcus aureus, Escherichia coli, Pseudomonas aeruginosa,* human parainfluenza virus I, metapneumovirus, enterovirus 71, coronavirus 229E, cytomegalovirus, Coxsackievirus A, *Bordetella pertussis,* chlamydia pneumoniae, *haemophilus influenzae, streptococcus salivarius, Streptococcus pneumoniae, Neisseria meningitidis, Mycobacterium tuberculosis,* human parainfluenza viruses 2 and 3, Epstein-Barr virus, Cryptococcus, *Aspergillus fumigatus, Aspergillus flavus, Candida albicans, Legionella pneumophila,* Boca Virus, herpes simplex virus 1, varicella zoster virus, *corynebacterium diphtheria, Lactobacillus bulgaricus, Moraxella catarrhalis, Staphylococcus epidermidis, Streptococcus pyogenes,* Pneumocystis, *Klebsiella pneumoniae,* etc.). The experimental results are shown in Figs. 8-9 (Fig. 8 shows the result of PCR reaction system A, and Fig. 9 shows the result of PCR reaction system B).

### Example 6. Joint detection of multiple pathogens, mutual interference between primers and probes

Due to the principle of complementary base pairing, dimers would form between primers and/or probes, but this probability was rare and could be ruled out at the beginning of the design. However, when multiple pathogens were jointly detected, there were many primers and probes, and dimers were prone to form between primers and primers, between probes and probes, or between primers and probes. To ensure the conservative property of the design (the conservative property was crucial to the accuracy of detection), and to consider the mutual interference between various primers and probes, the primers and probes needed to be carefully designed.

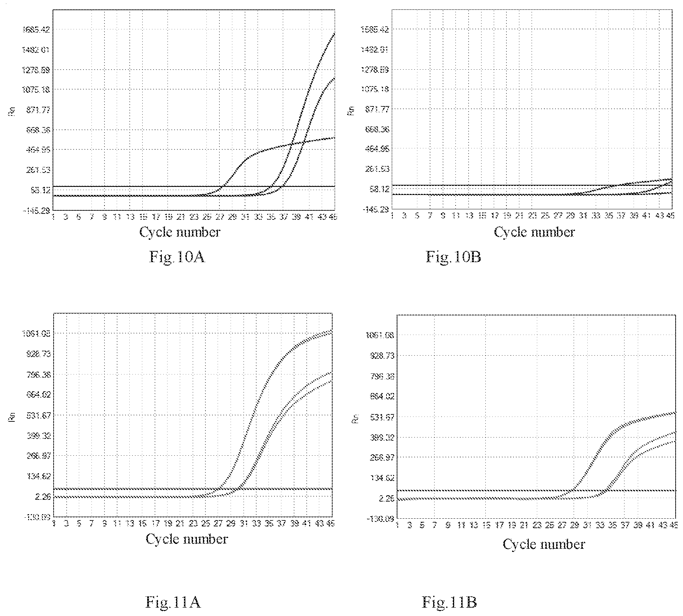

In the present application, the inventors replaced the respiratory syncytial virus in the present application with SARS-CoV-2, or replaced the rhinovirus in the present application with metapneumovirus. However, upon the experiments, it was found that the addition of SARS-CoV-2 and metapneumovirus would seriously affect the detection sensitivity of the method of the present application (experimental results are shown in Figs. 10-11).

## Claims

1. A composition for detecting and identifying a pathogen that causes a respiratory tract infection, comprising:
a first nucleic acid composition:
an influenza A forward primer as set forth in SEQ ID NO:1, an influenza A reverse primer as set forth in SEQ ID NO:2, and an influenza A probe as set forth in SEQ ID NO:3;
an influenza B forward primer as set forth in SEQ ID NO:4, an influenza B reverse primer as set forth in SEQ ID NO:5, and an influenza B probe as set forth in SEQ ID NO:6;
a rhinovirus forward primer as set forth in SEQ ID NO:7, a rhinovirus reverse primer as set forth in SEQ ID NO:8, a rhinovirus probe as set forth in SEQ ID NO:9, and a rhinovirus probe as set forth in SEQ ID NO:10; and
a second nucleic acid composition:
a respiratory adenovirus forward primer as set forth in SEQ ID NO: 11, a respiratory adenovirus forward primer as set forth in SEQ ID NO: 12, a respiratory adenovirus reverse primer as set forth in SEQ ID NO: 13, a respiratory adenovirus probe as set forth in SEQ ID NO: 14, and a respiratory adenovirus probe as set forth in SEQ ID NO: 15;
a respiratory syncytial virus forward primer as set forth in SEQ ID NO:16, a respiratory syncytial virus reverse primer as set forth in SEQ ID NO:17, and a respiratory syncytial virus probe as set forth in SEQ ID NO:18;
a mycoplasma pneumoniae forward primer as set forth in SEQ ID NO:19, a mycoplasma pneumoniae reverse primer as set forth in SEQ ID NO:20, and a mycoplasma pneumoniae probe as set forth in SEQ ID NO:21;
wherein, fluorescent groups comprised in the respective probes of the first nucleic acid composition are different from each other and do not interfere with each other, and fluorescent groups comprised in the respective probes of the second nucleic acid composition are different from each other and do not interfere with each other.

2. The composition of claim 1, wherein the composition further comprises an internal standard forward primer, an internal standard reverse primer and an internal standard probe, that are configured for monitoring.

3. The composition of claim 1, wherein the composition further comprises an internal standard forward primer as set forth in SEQ ID NO: 22, an internal standard reverse primer as set forth in SEQ ID NO: 23, and an internal standard probe as set forth in SEQ ID NO: 24.

4. The composition of claim 1, wherein a fluorescent reporter group of the influenza A probe as set forth in SEQ ID NO: 3 is FAM; a fluorescent reporter group of the influenza B probe as set forth in SEQ ID NO:6 is HEX; fluorescent reporter groups of the human rhinovirus probe as set forth in SEQ ID NOs: 9 and 10 are CY5.

5. The composition of claim 1, wherein a fluorescent reporter group of the respiratory adenovirus probe as set forth in SEQ ID Nos: 14 and 15 is FAM; a fluorescent reporter group of the respiratory syncytial virus probe as set forth in SEQ ID NO: 18 is HEX; a fluorescent reporter group of the mycoplasma pneumoniae probe as set forth in SEQ ID NO: 21 is CY5.

6. Use of the composition of any one of claims 1 to 5 in the preparation of a kit for detecting a pathogen that causes a respiratory tract infection.

7. A kit for detecting a pathogen that causes a respiratory tract infection and identifying the pathogen, comprising the first nucleic acid composition and the second nucleic acid composition of any one of claims 1 to 5 in separate packages.

8. The kit of claim 7, wherein the kit further comprises a nucleic acid release reagent and a PCR amplification system.

9. A method for detecting and identifying a pathogen that causes a respiratory tract infection, comprising:
a) releasing a nucleic acid in a sample to be detected;
b) performing a fluorescence quantitative PCR on the nucleic acid obtained in step 1) using the composition of any one of claims 1 to 5 or the kit of claim 7 or 8; and
c) obtaining a result and analyzing the result.

## Patentansprüche

1. Eine Zusammensetzung zum Nachweis und zur Identifizierung eines Erregers, der eine Atemwegsinfektion verursacht, aufweisend:
eine erste Nukleinsäurezusammensetzung:
einen Influenza-A-Vorwärtsprimer, wie in SEQ ID NO:1 dargelegt, einen Influenza-A-Rückwärtsprimer, wie in SEQ ID NO:2 dargelegt, und eine Influenza-A-Probe, wie in SEQ ID NO:3 dargelegt;
einen Influenza-B-Vorwärtsprimer, wie in SEQ ID NO:4 dargelegt, einen Influenza-B-Rückwärtsprimer, wie in SEQ ID NO:5 dargelegt, und eine Influenza-B-Probe, wie in SEQ ID NO:6 dargelegt;
einen Rhinovirus-Vorwärtsprimer, wie in SEQ ID NO:7 dargelegt, einen Rhinovirus-Rückwärtsprimer, wie in SEQ ID NO:8 dargelegt, eine Rhinovirus-Probe, wie in SEQ ID NO:9 dargelegt, und eine Rhinovirus-Probe, wie in SEQ ID NO:10 dargelegt; und
eine zweite Nukleinsäurezusammensetzung:
einen respiratorischen Adenovirus-Vorwärtsprimer, wie in SEQ ID NO: 11 dargelegt, einen respiratorischen Adenovirus-Vorwärtsprimer, wie in SEQ ID NO: 12 dargelegt, einen respiratorischen Adenovirus-Rückwärtsprimer, wie in SEQ ID NO: 13 dargelegt, eine respiratorische Adenovirus-Probe, wie in SEQ ID NO: 14 dargelegt, und eine respiratorische Adenovirus-Probe, wie in SEQ ID NO: 15 dargelegt;
einen Respiratory-Syncytial-Virus us-Vorwärtsprimer, wie in SEQ ID NO:16 dargelegt, einen Respiratory-Syncytial-Virus-Rückwärtsprimer, wie in SEQ ID NO:17 dargelegt, und eine Respiratory-Syncytial-Virus-Probe, wie in SEQ ID NO:18 dargelegt;
einen Mycoplasma pneumoniae-Vorwärtsprimer, wie in SEQ ID NO:19 dargelegt, einen Mycoplasma pneumoniae-Rückwärtsprimer, wie in SEQ ID NO:20 dargelegt, und eine Mycoplasma pneumoniae-Probe, wie in SEQ ID NO:21 dargelegt;
wobei die fluoreszierenden Gruppen, die in den jeweiligen Proben der ersten Nukleinsäurezusammensetzung enthalten sind, voneinander verschieden sind und sich nicht gegenseitig stören, und die fluoreszierenden Gruppen, die in den jeweiligen Proben der zweiten Nukleinsäurezusammensetzung enthalten sind, voneinander verschieden sind und sich nicht gegenseitig stören.

2. Die Zusammensetzung nach Anspruch 1, wobei die Zusammensetzung ferner einen internen Standard-Vorwärtsprimer, einen internen Standard-Rückwärtsprimer und eine interne Standardprobe umfasst, die zur Überwachung konfiguriert sind.

3. Die Zusammensetzung nach Anspruch 1, wobei die Zusammensetzung ferner einen internen Standard-Vorwärtsprimer, wie in SEQ ID NO: 22 dargelegt, einen internen Standard-Rückwärtsprimer, wie in SEQ ID NO: 23 dargelegt, und eine interne Standardprobe, wie in SEQ ID NO: 24 dargelegt, umfasst.

4. Die Zusammensetzung nach Anspruch 1, wobei eine fluoreszierende Reportergruppe der Influenza-A-Probe, wie in SEQ ID NO: 3 angegeben, FAM ist; eine fluoreszierende Reportergruppe der Influenza-B-Probe, wie in SEQ ID NO: 6 angegeben, HEX ist; fluoreszierende Reportergruppen der humanen Rhinovirus-Probe, wie in SEQ ID NOs: 9 und 10 angegeben, CY5 sind.

5. Die Zusammensetzung nach Anspruch 1, wobei eine fluoreszierende Reportergruppe der respiratorischen Adenovirus-Probe, wie in SEQ ID Nrn: 14 und 15 FAM ist; eine fluoreszierende Reportergruppe der respiratorischen Syncytialvirus-Probe, wie in SEQ ID NO: 18 dargelegt, HEX ist; eine fluoreszierende Reportergruppe der Mycoplasma pneumoniae-Probe, wie in SEQ ID NO: 21 dargelegt, CY5 ist.

6. Verwendung der Zusammensetzung nach einem der Ansprüche 1 bis 5 zur Herstellung eines Kits zum Nachweis eines Erregers, der eine Atemwegsinfektion verursacht.

7. Ein Kit zum Nachweis eines Erregers, der eine Atemwegsinfektion verursacht, und zur Identifizierung des Erregers, umfassend die erste Nukleinsäurezusammensetzung und die zweite Nukleinsäurezusammensetzung nach einem der Ansprüche 1 bis 5 in getrennten Packungen.

8. Der Kit nach Anspruch 7, wobei das Kit ferner ein Nukleinsäurefreisetzungsreagenz und ein PCR-Amplifikationssystem umfasst.

9. Ein Verfahren zum Nachweis und zur Identifizierung eines Erregers, der eine Atemwegsinfektion verursacht, umfassend:
a) Freisetzung einer Nukleinsäure in einer Probe, die nachgewiesen werden soll;
b) Durchführung einer quantitativen Fluoreszenz-PCR an der in Schritt 1) erhaltenen Nukleinsäure unter Verwendung der Zusammensetzung nach einem der Ansprüche 1 bis 5 oder des Kits nach Anspruch 7 oder 8; und
c) ein Ergebnis erhalten und das Ergebnis analysieren.

## Revendications

1. Composition de détection et d'identification d'un pathogène à l'origine d'une infection des voies respiratoires, comprenant :
une première composition d'acides nucléiques :
une amorce sens d'influenza A telle que présentée dans la SEQ ID NO : 1, une amorce antisens d'influenza A telle que présentée dans la SEQ ID NO : 2 et une sonde d'influenza A telle que présentée dans la SEQ ID NO : 3 ;
une amorce sens d'influenza B telle que présentée dans la SEQ ID NO : 4, une amorce antisens d'influenza B telle que présentée dans la SEQ ID NO : 5 et une sonde d'influenza B telle que présentée dans la SEQ ID NO : 6 ;
une amorce sens de rhinovirus telle que présentée dans la SEQ ID NO : 7, une amorce antisens de rhinovirus telle que présentée dans la SEQ ID NO : 8, une sonde de rhinovirus telle que présentée dans la SEQ ID NO : 9 et une sonde de rhinovirus telle que présentée dans la SEQ ID NO : 10 ; et
une seconde composition d'acides nucléiques :
une amorce sens d'adénovirus respiratoire telle que présentée dans la SEQ ID NO : 11, une amorce sens d'adénovirus respiratoire telle que présentée dans la SEQ ID NO : 12, une amorce antisens d'adénovirus respiratoire telle que présentée dans la SEQ ID NO : 13, une sonde d'adé-novirus respiratoire telle que présentée dans la SEQ ID NO : 14 et une sonde d'adénovirus respiratoire telle que présentée dans la SEQ ID NO : 15 ;
une amorce sens de virus respiratoire syncytial telle que présentée dans la SEQ ID NO : 16, une amorce antisens de virus respiratoire syncytial telle que présentée dans la SEQ ID NO : 17 et une sonde de virus respiratoire syncytial telle que présentée dans la SEQ ID NO : 18 ;
une amorce sens de *Mycoplasma pneumoniae* telle que présentée dans la SEQ ID NO : 19, une amorce antisens de *Mycoplasma pneumoniae* telle que présentée dans la SEQ ID NO : 20 et une sonde de *Mycoplasma pneumoniae* telle que présentée dans la SEQ ID NO : 21 ;
dans laquelle des groupes fluorescents compris dans les sondes respectives de la première composition d'acides nucléiques sont différents les uns des autres et n'interfèrent pas les uns avec les autres, et des groupes fluorescents compris dans les sondes respectives de la seconde composition d'acides nucléiques sont différents les uns des autres et n'interfèrent pas les uns avec les autres.

2. Composition selon la revendication 1, la composition comprenant en outre une amorce sens étalon interne, une amorce antisens étalon interne et une sonde étalon interne, qui sont conçues à des fins de surveillance.

3. Composition selon la revendication 1, la composition comprenant en outre une amorce sens étalon interne telle que présentée dans la SEQ ID NO : 22, une amorce antisens étalon interne telle que présentée dans la SEQ ID NO : 23 et une sonde étalon interne telle que présentée dans la SEQ ID NO : 24.

4. Composition selon la revendication 1, dans laquelle un groupe reporteur fluorescent de la sonde d'influenza A telle que présentée dans la SEQ ID NO : 3 est FAM ; un groupe reporteur fluorescent de la sonde d'influenza B telle que présentée dans la SEQ ID NO : 6 est HEX ; des groupes reporteurs fluorescents de la sonde de rhinovirus humain telle que présentée dans les SEQ ID NO : 9 et 10 sont CY5.

5. Composition selon la revendication 1, dans laquelle un groupe reporteur fluorescent de la sonde d'adénovirus respiratoire telle que présentée dans les SEQ ID NO : 14 et 15 est FAM ; un groupe reporteur fluorescent de la sonde de virus respiratoire syncytial telle que présentée dans la SEQ ID NO : 18 est HEX ; un groupe reporteur fluorescent de la sonde de *Mycoplasma pneumoniae* telle que présentée dans la SEQ ID NO : 21 est CY5.

6. Utilisation de la composition selon l'une quelconque des revendications 1 à 5 dans la préparation d'une trousse de détection d'un pathogène à l'origine d'une infection des voies respiratoires.

7. Trousse de détection d'un pathogène à l'origine d'une infection des voies respiratoires et d'identification du pathogène, comprenant la première composition d'acides nucléiques et la seconde composition d'acides nucléiques selon l'une quelconque des revendications 1 à 5 dans des conditionnements séparés.

8. Trousse selon la revendication 7, la trousse comprenant en outre un réactif de libération d'acide nucléique et un système d'amplification par PCR.

9. Procédé de détection et d'identification d'un pathogène à l'origine d'une infection des voies respiratoires, comprenant :
a) la libération d'un acide nucléique dans un échantillon à détecter ;
b) la réalisation d'une PCR quantitative par fluorescence sur l'acide nucléique obtenu à l'étape 1) à l'aide de la composition selon l'une quelconque des revendications 1 à 5 ou de la trousse selon la revendication 7 ou 8 ; et
c) l'obtention d'un résultat et l'analyse du résultat.
